Europäisches Patentamt

European Patent Office

Office européen des brevets

(19)

(11) EP 1 275 404 A1

(12) **EUROPEAN PATENT APPLICATION**

(43) Date of publication:
**15.01.2003 Bulletin 2003/03**

(51) Int Cl.7: **A61L 15/46**, C08B 37/00

(21) Application number: **01202668.8**

(22) Date of filing: **11.07.2001**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **SCA Hygiene Products AB
405 03 Göteborg (SE)**

(72) Inventors:
• **Besemer, Arie Cornelis
3958 CE Amerongen (NL)**

• **Thiewes, Harm Jan
3931 CC Woudenberg (NL)**

(74) Representative: **Jorritsma, Ruurd et al
Nederlandsch Octrooibureau
Scheveningseweg 82
P.O. Box 29720
2502 LS Den Haag (NL)**

(54) **Odour regulation in hygiene products**

(57)    Oxidised cyclodextrins containing an average of at least 0.5 carboxyl group and/or at least 0.5 functional group selected from aldehyde groups and aldehyde-derived groups per molecule can be used as complexing agents, in particular in odour control for hygiene products. A fragrance component can be incorporated in the oxidised cyclodextrin and released when in use, and, in addition or alternatively, the oxidised cyclodextrin can absorb undesired odour components during use.

EP 1 275 404 A1

**Description**

The invention relates to odour regulation in hygiene products using oxidised compounds capable of absorbing odorous components and of releasing odour-regulating compounds.

It is known from JP-A-1-015049 to add cyclodextrins to paper diapers for the purpose of deodorising the paper diaper. A fragrance is included in the cyclodextrin and can be released in the diaper, while absorption of malodours by the non-included cyclodextrin adds to the deodorising effect. Similarly, WO 94/22501 describes articles containing cyclodextrin having a particle size below 12 μm, especially below 5 μm, for removal of odour from diapers and paper towels. On the other hand, cyclodextrin particles of greater than 12 μm, typically around 100 μm, are said to be effective for odour control in absorbent articles according to WO 99/06078. WO 00/66187 discloses odour-controlled superabsorbent polymers containing cyclodextrins or cyclodextrin ethers such a methyl- or hydroxyalkyl-cyclodextrins homogeneously distributed therein.

While cyclodextrins have been known for a long time as being useful for inclusion of a variety of agents, including odour-regulating agents, the most important member thereof, β-cyclodextrin, has a poor solubility and hence a difficult applicability in various product types, such as hygiene products.

A novel type of oxidation products has been found now, which are cyclic oligomers consisting of glucose, glucuronic acid and glucuraldehyde units. The products to be used according to the invention are obtained by oxidising cyclodextrins, and contain at least 1 carboxyl group or carboxyl-derived group, aldehyde group and/or aldehyde-derived group per cyclodextrin molecule are very suitable as odour-regulating agents in hygiene products and tissue products.

Cyclodextrins according to the invention comprise cyclooligomers of anhydroglucose units (AGU), having at least 6 AGU (α-cyclodextrin), preferably 7 AGU (β-cyclodextrin), possibly 8 (γ-cyclodextrin) or more AGU (δ- and higher cyclodextrins). As used herein, carboxyl-derived group means salts and hydrolysable esters of the carboxylic acid, and aldehyde-derived group means especially nitrogen-based and oxygen-based derivatives of aldehydes. Nitrogen-based derivatives of aldehydes include products obtained by amination or reductive amination of the aldehyde group. Oxygen-based derivatives include products obtained by acetalisation of the aldehyde group. Nonlimiting examples are:

- amines obtained by reductive amination having the formula:

$$-CH_2-NH-R;$$

- oximes obtained by reaction with hydroxylamines or ethers thereof, having the formula: -CH=N-OR;
- hydrazines having the formula: $-CH_2-NH-NH-R$;
- hydrazones having the formula: -CH=N-NH-R;
- hydrazides having the formula: -CH=N-NH-CO-R; wherein R in these formulas may be hydrogen, alkyl, e.g. $C_1-C_{20}$ alkyl, cycloalkyl, aryl, or combinations thereof such as aralkyl;
- hemiacetals and acetals having the formula $-CH(OR^3)-OR^2$ or $-CH(-OR^2)_2$, in which $R^2$ is alkyl or carboxymethyl, $R^3$ is hydrogen or a direct bond to the oxygen atom of a dehydrogenated hydroxyl group of the carbohydrate (forming a lactone).

In a particular embodiment, the aldehyde-derived groups are hydrophobic groups of the formula $-CH_2-NH-R$ or any other of the formulae above, wherein R is long-chain ($C_6-C_{20}$, especially $C_{12}-C_{18}$) alkyl, alkenyl or aralkyl. These derivatives can be used to modify the solubility or the surface-activity of the oxidised cyclodextrins and their complexes.

The oxidised cyclodextrins of the invention can be prepared by selective oxidation of the 6-hydroxymethyl group of the anhydroglucose units of the cyclodextrins, e.g. using nitrogen dioxide or by nitroxyl-mediated oxidation. The preferred oxidation uses TEMPO (2,2,6,6-tetramethylpyridin-1-oxyl) or analogous di-tertiary and/or cyclic nitroxyl compounds. Suitable examples of TEMPO analogues include 4,4-dimethyloxazolidine-N-oxyl (DOXYL), 2,2,5,5-tetramethylpyrrolidine-N-oxyl (PROXYL), 4-hydroxy-TEMPO and esters and other derivatives and polymers thereof, 4-acetamido-TEMPO, and the like. Usually a co-oxidant is used for producing the nitroxyl or nitrosonium active species. Such a co-oxidant may be hypohalite, or a peracid, such as peracetic acid, perbenzoic acid or persulphuric acid. The nitroxyl can be used in a catalytic amount only. The catalytic amount of nitroxyl is preferably 0.1-20% by weight, based on the cyclodextrin, or 0.1-20 mol% with respect to the cyclodextrin, preferably 0.5-5% by weight.

TEMPO-mediated oxidation of carbohydrates is described e.g. in WO 95/07303 and WO 99/57158. Oxidation using nitrosonium ions derived from TEMPO and analogues is described in WO 00/50388 and WO 00/50621. This latter type of oxidation, wherein an oxidative enzyme, such as a laccase, or a metal complex, such as copper/bipyridyl, is used with oxygen or hydrogen peroxide as a co-oxidant was also found to very suitable for the oxidation of cyclodextrins. Oxidation of β-cyclodextrin and other poly- and oligosaccharides with sodium nitrate in concentrated phosphoric acid using sodium nitrite as a catalyst is described in NL 9301172. Oxidation of cyclodextrins using TEMPO and hypochlorite/ bromide was described by Chang and Robyt (*Carbohydrate Lett.* **3** (1998) 31-38), and by Fraschini and Vignon (*Carbohydrate Res.* **328** (2000) 585-589), but only reported to give carboxylic acid derivatives, which have increased solubility. Yoon et al. (*J. Org. Chem.* **60** (1995) 2792-2795) described the synthesis of cyclodextrin aldehydes by DMSO oxidation of the tosylates, and the conversion thereof to carboxylic acids, oxime, hydrazone and phenylamine derivatives.

The process of the invention results in oxidation of the primary hydroxyl groups (at the 6-positions) initially to the corresponding aldehydes, and eventually to the corresponding carboxylic acids. In general, the second oxidation step, from aldehyde to carboxylic acid, proceeds at a higher rate than the first step, i.e. the oxidation from alcohol to aldehyde. It was found that the relative amount of aldehyde groups is higher during the first stages of the oxidation; depending on the particular nitroxyl compound, and the further reaction parameters, the relative amount of carboxyl groups becomes higher after 10-60% oxidation (see also table 3, below). The oxidised cyclodextrin thus obtained preferably contains an average of at least 1.0 functional groups selected from carboxyl groups, aldehyde groups, and aldehyde-derived groups per molecule. The total number of functional groups is preferably between 1.5 and 3 per molecule for $\alpha$-cyclodextrin derivatives (cyclomaltohexaose), between 1.5 and 4 for $\beta$-cyclodextrin derivatives and between 1.5 and 5 for $\gamma$-cyclodextrin derivatives and higher homologues. More specifically, the oxidised cyclodextrin contains at least 0.5 carboxyl group per molecule and/or at least 0.5 aldehyde or aldehyde-derived group per molecule. Further adjustment of the ration of aldehyde to acid can be achieved by reduction of the aldehyde groups with reducing agents such as $NaBH_4$ or by oxidation with oxidizing agents such as $NaClO_2$.

It was found that the ratio of aldehyde to carboxylic acid groups can be selected by selecting the oxidation parameters, and by selecting the particular nitroxyl derivative. For example, relatively high levels of aldehyde are obtained when performing the oxidation using 4-acetamido TEMPO, whereas 4-acetoxy TEMPO results in the highest amounts of carboxylic acid. 4-Acetamido-TEMPO has the further advantage of allowing a homogeneous oxidation reaction in aqueous medium.

The primary oxidised product is usually a mixture of cyclodextrins having varying numbers of carboxyl and aldehyde groups. If needed, the non-oxidised cyclodextrins, which can be identified using HPLC, are separated from the oxidised compounds by column chromatography, preferably using a weakly anionic ion-exchange resin. The products have an improved water solubility of at least 50 g/l.

Another group of compounds according to the invention having both aldehyde and carboxylic acid functions can be obtained by oxidising the cyclodextrin with periodate, followed by oxidation of the intermediate dialdehyde with peracetic acid and bromine, as described in WO 00/26257. This method results in about half of the aldehyde groups of the dialdehyde groups being further oxidised to carboxyl groups; this product can be referred to as mono-aldehyde-carboxylic acid cyclodextrin (MAC-CD). This product contains at least one unit (and preferably only one unit) of the formulas [-O-CH(COOH)-CH(CH$_2$OH)-O-CH(CHO)-] or [-O-CH(CHO)-CH(CH$_2$OH)-O-CH(COOH)-].

The aldehyde products of the invention can be further functionalised by reaction with suitable nitrogen-containing reagents, such as primary amines, hydroxylamines, hydrazine, and the like, as described above. The aldehyde products have the further advantage of directly binding ammonia and amine compounds, e.g. from urine in a hygiene article.

A surprising feature of the compounds of the invention is that inclusion complexes of cyclodextrins having a high degree of oxidation do not precipitate from an aqueous solution under neutral and alkaline conditions, and that the complex can be precipitated after protonation.

The products of the invention are especially useful as an odour regulator in hygiene products, such as diapers, napkins and tissue products such as wipes for kitchen rolls and facial tissues, bathroom towels etc., by scavenging malodours. They can also assist in controlling bacterial growth, resulting in reduced ammonia production e.g. in diapers. They can be incorporated into such products in an amount varying between 1 and 100 mg/g. The odour regulation can be effected in two ways. Firstly, the oxidised cyclodextrins serve to absorb odorous components from the fluid or solid material for which the hygiene material is used, such as sulphur compounds, amines, carbonyl compounds, aromatic compounds and the like. Secondly, a desired neutralising odour or fragrance may be incorporated in the product prior to its use, and can be released or exchanged in use, resulting in a neutral and/or pleasant odour in the product in use. Examples of suitable fragrances include terpenoid compounds such as linalool, menthone, menthol, limonene and pinene.

Other applications are in the pharmaceutical and medical field, to enhance the solubility of hydrophobic drugs, and/or to improve the smell, taste or irritation potential of the drugs, or in biocides, for odour control, stabilisation of encapsulated compounds to light, oxidation and vaporisation, slow release, and in enantiomer separation.

**Examples.** *General*

The TEMPO-mediated oxidation of primary alcohols is known to be selective over the oxidation of the secondary groups. The first oxidation step leads to aldehydes and consecutive reaction to carboxylic acids. The amount of aldehydes can be derived from the sodium hydroxide consumption, which corresponds to carboxylic acid formation. For the formation of one aldehyde group one mol of hypochlorite is needed, whereas the formation of acid requires two mols. Based on these assumptions the total degree of oxidation can be derived. The total degree of oxidation is the ratio between the sum of aldehyde and carboxylic acid groups and the number of primary alcohol groups available to be oxidised. Note that for full oxidation of oneprimary hydroxyl groups 2 mmol of hypochlorite would be needed, following the equation:

$$R\text{-}CH_2OH + 2\ HOCl \rightarrow R\text{-}COOH + H_2O + 2\ HCl$$

The amount of uronic acids can be determined according to the method described by Blumenkrantz and Asboe (N. Blumenkrantz and G. Asboe-Hansen, *Anal. Biochem.* **54** 484 (1973)). Aldehydes can be determined by titration of the hydrochloric released upon reaction of aldehydes with hydroxylamine hydrochloric acid.

**Example 1.**

To a solution of 300 mg NaBr and 50 mg of TEMPO in 200 ml water, 2.22 g β-CD (CAVAMAX® W7 from Wacker-CHEMIE GmbH, 1.95 g on dry weight basis = 12 mmol) was added. During standing a precipitate was formed (complex of TEMPO and CD). After 15 minutes stirring, 0.2 ml solution of sodium hypochlorite (2.04 M) was added. During reaction the pH tends to decrease due to the formation of acid. The pH was kept at pH 9.5 by addition of sodium hydroxide solution (0.5M), controlled by a pH stat (Metrohm). After the reaction rate decreased, additional hypochlorite was added and this was repeated until the desired amount of sodium hypochlorite (5.2 ml corresponding to 10.7 mmol) was added. The mixture was allowed to react until no further sodium hydroxide was consumed. The pH of the mixture was brought to 4.0 by addition of diluted sulphuric acid, followed by 150 mg hydroxylamine hydrochloride (Merck). The pH started to drop again, due to the formation of oximes. To neutralise the hydrochloric acid 2.35 ml 0.5 M NaOH was needed.
It was found that aldehyde formation takes place especially in the initial phase. In the later phase, a steady-state reaction is observed where aldehyde formation and reaction of the aldehyde to carboxylate are in equilibrium. At this stage, the production of acid vs. consumption of HOCl was found to be 0.48 mmol. Table 3, below, shows the relative amounts of aldehyde and carboxylate. According to the method for the determination of uronic acids (Asboe and Blumenkrantz, *see above)* 1.02 g of uronic acid was formed. This corresponds to a degree of oxidation of 5.7 mmol of uronic acid. Since the amount of aldehyde was found to be 1.2 mmol, the total degree of conversion was 6.9 mmol (57 %). The material obtained readily formed precipitates with toluene and menthone, respectively.

**Example 2.**

The experiment was repeated, however, with the aim to reach a higher degree of oxidation. With the same amounts of CD, NaBr and TEMPO, 8 ml HOCl (16.4 mmol) was used. The production of acid as a function of hypochlorite consumption in the steady state was in agreement with the value found in Example 1 (0.47 mmol/mmol). The calculated and experimentally determined degrees of oxidation were also in good agreement. (75 and 70 %, respectively). The material obtained formed a precipitate with toluene and menthone, respectively only when a concentrated solution was used (approximately 2g per 10 ml), which indicates that the complex is better soluble in water than the cyclodextrin with a lower degree of oxidation.

Table 1.

| Adsorption of phenolphthalein by oxidised β-cyclodextrin | | | | |
|---|---|---|---|---|
| Theoretical DO | DO (determined) Blumenkrantz[1] | Phenolphthalein Absorption (at λ=553 nm)[2] | Toluene inclusion[3] | Menthone inclusion[3] |
| 0 | -- | 0.025 | Yes | Yes |
| 1/7 | 10 | ------ | Yes | Yes |
| 2/7 | 18 | 0.068 | Yes | Yes |

[1] According to: *New method for quantitative determination of uronic acids*, Analytical Biochemistry **54,** (1973) 484-489.

2) Sample concentration of 1mg/ml sodium carbonate buffer pH 10.3 mixed with 10μl 0.1% phenolphthalein in ethanol.

3) 100 mg of sample dissolved in water and 20 μl toluene or menthone added to notice precipitation.

Table 1. (continued)

| Adsorption of phenolphthalein by oxidised β-cyclodextrin | | | | |
|---|---|---|---|---|
| Theoretical DO | DO (determined) Blumenkrantz[1] | Phenolphthalein Absorption (at λ=553 nm)[2] | Toluene inclusion[3] | Menthone inclusion[3] |
| 3/7 | 22 | 0.156 | Yes | Yes |

[1] According to: *New method for quantitative determination of uronic acids*, Analytical Biochemistry **54,** (1973) 484-489.

2) Sample concentration of 1mg/ml sodium carbonate buffer pH 10.3 mixed with 10μl 0.1% phenolphthalein in ethanol.

3) 100 mg of sample dissolved in water and 20 μl toluene or menthone added to notice precipitation.

**Example 3.**

Five grams of β-cyclodextrin (CAVAMAX® W7 from Wacker-CHEMIE GMBH) on dry weight basis was dissolved in 300 ml of demi-water, and 22.4 ml of a 0.032 M 4-acetamido TEMPO solution was added. Subsequently, β-cyclodextrin was oxidised by addition of sodium hypochlorite. Batches were prepared with theoretically one, two and three anhydroglucose units fully oxidised per cyclodextrin molecule (average) per β-cyclodextrin molecule using sodium hypochlorite. The pH was kept constant during reaction by addition of 0.5 M NaOH, controlled by a pH stat (Metrohm). After completion of the oxidation, the aldehyde groups were reduced with sodium borohydride.
The respective batches were concentrated in a rotary evaporator, and subsequently freeze-dried. The freeze-dried material was washed in a methanol/water mixture to remove salts and 4-acetamido-TEMPO. The batches were finally washed with acetone and dried in an oven at 50°C. The inclusion properties were investigated (see Table1).

**Example 4.**

Ten grams of β-cyclodextrin (CAVAMAX® W7 from Wacker-CHEMIE GMBH) on dry weight basis were dissolved in 600 ml of demi-water, and 300 mg 4-acetamido TEMPO and 250 mg solution were added. The substrate β-cyclodextrin was oxidised, by addition of small amounts of sodium hypochlorite (0.5 ml 2.14 M). The reaction was conducted at room temperature and at pH 9.5. After each addition the pH started to decrease. When no further drop was observed, the next aliquot of hypochlorite was added. The total amount was chosen in such a way that one (samples A) or two (sample B) glucose units per β-cyclodextrin molecule could be oxidised After completion of the oxidation the sample was divided in two equal parts and aldehyde groups left were further modified (1) by further oxidation with sodium chlorite/hydrogen peroxide at pH 5 at 20 °C (yielding samples A1 and B1) or (2) by treatment with hydroxylamine at pH 3.2 at 20 °C yielding samples A2 and B2. According to titration with NaOH the first sample (A2) required 3.2 mmol to neutralize the released amount of HCl. The second batch (B2) needed 4.35 mmol.

Table 2.

| Adsorption of phenolphthalein by oxidised β-cyclodextrin | | | | |
|---|---|---|---|---|
| Sample | DO[1] | Phenolphthalein Absorption (at λ=553 nm)[2] | Toluene inclusion[3] | Menthone inclusion[3] |
| 0 | -- | 0.025 | Yes | Yes |
| A1 | 15 | 0.021 | Yes | Yes |
| A2 | 9 | 0.011 | Yes | Yes |
| B1 | 22 | 0.153 | Yes | Yes |
| B2 | 14.5 | 0.009 | Yes | Yes |

[1] According to: *New method for quantitative determination of uronic acids*, Analytical Biochemistry **54,** (1973)484-489.

2) Sample concentration of 1mg/ml sodium carbonate buffer pH 10.3 mixed with 10μl 0.1% phenolphthalein in ethanol. A solution in which no cyclodextrin is present has E = 0.65

3) 100 mg of sample dissolved in water and 20 μl toluene or menthone added to notice precipitation.

In case of route 1) samples were concentrated in a rotary evaporator, and subsequently freeze-dried. The freeze-dried material was washed in a methanol/water mixture to remove salts and 4-acetamido TEMPO. The samples were further washed with acetone and dried in a vacuum oven at room temperature.
In case of route 2) samples were concentrated in a rotary evaporator, and part of the product was precipitated by addition of ethanol (water-ethanol is 1:2). Liquid phase and precipitate were separated by a glass filter and the precipitate was washed with acetone, and subsequently dried in a vacuum oven at room temperature. Modified cyclodextrins were obtained with the characteristics as mentioned in table 2.

**Example 5**

A solution was prepared of 7.64 g β-cyclodextrin, 150 mg NaBr and 150 mg 4-acetamido TEMPO in 300 ml water. Then sodium hypochlorite was added in doses of 0.200 ml per time. After each dose the reaction was allowed to proceed until no further NaOH consumption was seen. During reaction the pH was kept at 9.3 by addition of NaOH controlled by a pH stat.
The content of the aldehyde and carboxylic acid after 25 % oxidation was, within experimental error, in agreement with the calculated values (7 mmol aldehyde and 7 mmol uronic acid). After 50 % oxidation we found that 23 mmol acid and 6 mmol aldehyde was present ( see also table 3, below).

**Example 6**

The experiment as described in Example 1 was repeated, however using 4-acetoxy TEMPO (300 mg) instead of TEMPO. The amount of sodium hypochlorite was 8.0 mmol. Table 3 summarises the results of the various experiments with different TEMPO-analogues with regard to formation of aldehydes and acids.

Table 3.

| *Aldehyde and acid-formation (%) upon oxidation of cyclodextrin using different TEMPO-analogues* | | | | | | | | |
|---|---|---|---|---|---|---|---|---|
| | 4-acetamido TEMPO | | | 4-acetoxy-TEMPO | | | TEMPO | | |
| | aldehyde | acid | total | aldehyde | acid | total | aldehyde | acid | Total |
| 25%NaOCl | 30 | 10 | 40 | 12 | 20 | 32 | 15 | 18 | 33 |
| 50%NaOCl | 34 | 33 | 67 | n.d | n.d. | n.d. | 16 | 38 | 54 |
| n.d.= not determined | | | | | | | | | |

**Example 7**

From 4.7 g cyclodextrin (dry) an oxidised cyclodextrin was prepared as described in example 1, using 10ml 2.0 M NaOCl solution, 150 mg NaBr and 80 mg 4-acetamido-TEMPO. The ratio acid/aldehyde was derived from the sodium hydroxide consumption and experimentally confirmed. According to calculation, 6.3 mmol acid and 6.9 mmol aldehyde were produced. By titration with hydroxylamine hydrochloride, it was found that 5.7 mmol aldehyde was formed and according to the uronic acid assay 8.0 mmol acid was formed, somewhat higher than calculated (6-aldehyde polysaccharides contribute to a limited extent to the response in the uronic acid assay). The reaction mixture was concentrated in vacuum to about 120 ml. To 5 ml of this solution 40 μl toluene was added and this mixture was stirred vigorously for one minute. Precipitation of the oxidised cyclodextrin-toluene complex started slowly i.e. after 10 minutes it was visible that a precipitate was formed. After 5 hours the process of precipitation formation seemed to be finished. The same experiment was repeated with an acidified solution. (pH 3 ) Here precipitation started immediately after mixing. The process was complete within one hour.

**Example 8**

From 4.7 g cyclodextrin (dry) an oxidised cyclodextrin was prepared as described in example 1, using 5 ml 2.0 M NaOCl solution, 150 mg NaBr and 4-acetamido-TEMPO. The ratio acid/aldehyde was derived from the sodium hydroxide consumption and experimentally confirmed. According to calculation 2.2 mmol acid and 5.7 mmol aldehyde were produced. By titration with hydroxylamine hydrochloride it was found that 6.0 mmol aldehyde was formed and according to the uronic acid assay 3.5 mmol acid was formed (higher than calculated). The reaction mixture was concentrated in vacuum to about 120 ml. To 5 ml of this solution 40 μl toluene was added and this mixture was stirred vigorously for one minute. Precipitation of the oxidised cyclodextrin-toluene complex started immediately and after one hour the process of precipitation formation appeared to be finished.

**Example 9**

In 150 ml of a buffered solution in water (4 mM sodium acetate/acetic acid, pH 5.6) 1.78 g cyclodextrin, 120 mg 4-acetamido TEMPO and 20 mg laccase (crude material obtained from *Trametes versicolor)* were dissolved. This solution was kept at 38 °C. Through the reaction mixture oxygen gas was led. After a few hours of reaction the pH started to decrease. After 4 hours another batch of laccase (15 mg) was added and this was repeated after 20 hours reaction. The pH was maintained between 5 and 6 by adding 0.5 M NaOH. After 24 hours 2.0 ml 0.5 M NaOH was consumed. According to the Blumenkrantz assay 225 mg uronic acid was formed. By titration with hydroxylamine hydrochloride, it was established that 0.75 mmol aldehyde was formed. Based on uronic acid and titration, it appears that the total degree of conversion is 2.05 mmol (18 % conversion). This material formed readily a precipitate with toluene.

**Claims**

1. Use of an oxidised cyclodextrin containing an average of at least 1.0 functional group selected from carboxyl groups, aldehyde groups, and aldehyde-derived groups per molecule, as an odour regulator in hygiene products.

2. Use according to claim 1, wherein the oxidised cyclodextrin contains between 1.5 and 4 functional groups per molecule.

3. Use according to claim 1 or 2, having at least 0.5 carboxyl group per molecule.

4. Use according to any one of claims 1-3, having at least 0.5 aldehyde or aldehyde-derived group per molecule.

5. Use according to any one of claims 1-4, wherein a fragrance is incorporated in said oxidised cyclodextrin.

6. An odour-controlled cellulosic tissue material, comprising between 0.1 and 10% by weight of an oxidised cyclodextrin as defined in any one of the preceding claims.

7. A tissue material according to claim 6, wherein the material is a hygiene product.

8. An oxidised cyclodextrin containing an average of at least 0.5 carboxyl group and at least 0.5 functional group selected from aldehyde groups and aldehyde-derived groups per molecule.

9. An oxidised cyclodextrin according to claim 8, which is an oxidised β-cyclodextrin.

10. An oxidised cyclodextrin according to claim 8 or 9, which is essentially free of non-oxidised cyclodextrin.

11. A process for producing an oxidised cyclodextrin to be used according to any one of claims 6-8, comprising reacting cyclodextrin with an oxidising agent in the presence of a catalytic amount of a nitroxyl compound.

12. A process according to claim 11, wherein the nitroxyl compound is a 4-acylamino-TEMPO.

13. Use of an oxidised cyclodextrin according to claim 8 or 9, as a slow-release agent.

**European Patent Office**

**EUROPEAN SEARCH REPORT**

Application Number

EP 01 20 2668

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | CHANG P S ET AL: "OXIDATION OF THE PRIMARY ALCOHOL GROUPS OF CYCLOMALTODEXTRINS WITH 2,2,6,6-TETRAMETHYL-1-PIPERIDINE OXOAMMONIUM ION" CARBOHYDRATE LETTERS, HARWOOD ACADEMIC PUBLISHERS, BASEL, CH, vol. 3, 1998, pages 31-38, XP000775568 ISSN: 1073-5070 | 11,13 | A61L15/46 C08B37/00 |
| Y | * abstract * * page 38, paragraph 1 * | 11,12 | |
| Y | WO 01 34657 A (SCA HYGIENE PRODUCTS ZEIST B V ;GUNNARS SUSANNA (SE)) 17 May 2001 (2001-05-17) * the whole document * | 11,12 | |
| X | WO 01 48025 A (KIMBERLY CLARK CO) 5 July 2001 (2001-07-05) * claims 1,15-17 * | 1,6 | |
| E | WO 01 55222 A (DUVAL RAPHAEL ;VIGNON MICHEL (FR); CHIRALSEP SARL (FR); FRASCHINI) 2 August 2001 (2001-08-02) * claims 1,4 * | 11,13 | TECHNICAL FIELDS SEARCHED (Int.Cl.7) A61L A61F C08B |
| A | LINQI SHI ET AL: "NOVEL COMPOSITE ADSORBENT FOR ADSORPTION OF UREA" POLYMERS FOR ADVANCED TECHNOLOGIES, JOHN WILEY AND SONS, CHICHESTER, GB, vol. 10, no. 1/2, January 1999 (1999-01), pages 69-73, XP000799439 ISSN: 1042-7147 * abstract * | 1,8,13 | |

-/--

The present search report has been drawn up for all claims

| Place of search | Date of completion of the search | Examiner |
|---|---|---|
| THE HAGUE | 19 December 2001 | Muñoz, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 01 20 2668

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,D | FRASCHINI C ET AL: "Selective oxidation of primary alcohol groups of beta-cyclodextrin mediated by 2,2,6,6-tetramethylpiperidine-1-oxyl radical (TEMPO)" CARBOHYDRATE RESEARCH, ELSEVIER SCIENTIFIC PUBLISHING COMPANY. AMSTERDAM, NL, vol. 328, no. 4, 6 October 2000 (2000-10-06), pages 585-589, XP004218900 ISSN: 0008-6215 * abstract * | 8 | |
| A | CORNWELL M J ET AL: "A One-Step Synthesis of Cyclodextrin Monoaldehydes" TETRAHEDRON LETTERS, ELSEVIER SCIENCE PUBLISHERS, AMSTERDAM, NL, vol. 36, no. 46, 13 November 1995 (1995-11-13), pages 8371-8374, XP004026914 ISSN: 0040-4039 * abstract * | 8 | |
| A | EP 1 077 221 A (NAT STARCH CHEM INVEST) 21 February 2001 (2001-02-21) * the whole document * | 11,12 | TECHNICAL FIELDS SEARCHED (Int.Cl.7) |

The present search report has been drawn up for all claims

| Place of search | Date of completion of the search | Examiner |
|---|---|---|
| THE HAGUE | 19 December 2001 | Muñoz, M |

EPO FORM 1503 03.82 (P04C01)

EP 1 275 404 A1

**ANNEX TO THE EUROPEAN SEARCH REPORT
ON EUROPEAN PATENT APPLICATION NO.**          EP 01 20 2668

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2001

| Patent document cited in search report | | Publication date | Patent family member(s) | | Publication date |
|---|---|---|---|---|---|
| WO 0134657 | A | 17-05-2001 | AU | 1741101 A | 06-06-2001 |
| | | | WO | 0134657 A1 | 17-05-2001 |
| WO 0148025 | A | 05-07-2001 | AU | 2593501 A | 09-07-2001 |
| | | | WO | 0148025 A1 | 05-07-2001 |
| WO 0155222 | A | 02-08-2001 | FR | 2804437 A1 | 03-08-2001 |
| | | | AU | 3195101 A | 07-08-2001 |
| | | | WO | 0155222 A1 | 02-08-2001 |
| EP 1077221 | A | 21-02-2001 | AU | 5340700 A | 22-02-2001 |
| | | | BR | 0003645 A | 27-03-2001 |
| | | | CN | 1298885 A | 13-06-2001 |
| | | | EP | 1077221 A1 | 21-02-2001 |
| | | | JP | 2001122904 A | 08-05-2001 |